# EUROPEAN PATENT APPLICATION

(11) **EP 3 968 121 A1**
(43) Date of publication of application: **16.03.2022**
(21) Application number: 20802187.3
(22) Date of filing: 17.04.2020
(51) Int. Cl.: G05D 27/02

(54) **METHOD, SYSTEM AND APPARATUS FOR ADJUSTING SLEEP ENVIRONMENT**

(30) Priority: 09.05.2019 HK 19123551
(71) Applicant: Liao, Chun Zu Joe, Yau Yat Chuen, Kowloon (HK)
(72) Inventor: Liao, Chun Zu Joe, Yau Yat Chuen, Kowloon (HK)
(74) Representative: Cabinet Chaillot
(86) International application number: PCT/IB2020/053649
(87) International publication number: WO 2020/225626

(57) **Abstract**

Disclosed are a method, system and apparatus for adjusting a sleep environment. The method comprises the following steps: a data acquisition structure (10) acquiring data of user sleep and sending same to a data analysis server (11) (S1); the data analysis server (11) analyzing and processing the received data of the user sleep to obtain sleep information of a user and environment information of a sleep environment of the user, and sending the sleep information of the user and the environment information of the sleep environment of the user to an adjusting server (12) (S3); the adjusting server (12) generating, according to the received sleep information of the user and environment information of the sleep environment of the user, a corresponding environment control instruction, and sending the environment control instruction to a local server (13) (S15); and an adjustor (14) controlling and regulating, according to the received environment control instruction, the illumination intensity of an LED lamp (15), the volume of an audio player (16) and the rotational speed of a fan (17) (S9).

## Description

### [TECHNICAL FIELD]

The present invention relates to the technical field of environmental adjustment, particularly to a method, a system and an apparatus for adjusting a sleep environment.

### [BACKGROUND ART]

At present, the demand for accommodation in some places with restricted or limited space is increasing. For example, in densely populated metropolises like Hong Kong, Tokyo and Beijing, or places with high demand for short stay, such as airports and railway stations, cheap and temporary accommodation for sleep is needed normally. The accommodation places on the one hand should ensure sufficient privacy and on the other hand does not take up a large space. Therefore, rest devices have emerged.

The current rest devices are only able to provide users with places of sleep, but cannot adjust the sleep environment to ensure the comfort of the users' sleep and realize the high-quality sleep of the users. Therefore, an improved solution is urgently needed.

### [SUMMARY OF THE INVENTION]

An object of the present invention is to overcome the foregoing technical defects and provide a method, a system and an apparatus for adjusting a sleep environment, which can adjust the sleep environment of a user, thereby ensuring the comfort of the user's sleep and realizing the high-quality sleep of the user.

According to the first aspect of the present invention, a method for adjusting a sleep environment is provided, comprising the following steps:
a data acquisition structure acquiring data of user sleep and sending same to a data analysis server, the data of the user sleep comprising sleep data of a user and environment data of a sleep environment of a user;
the data analysis server analyzing and processing the received data of the user sleep to obtain sleep information of a user and environment information of a sleep environment of the user, and sending the sleep information of the user and the environment information of the sleep environment of the user to an adjusting server;
the adjusting server generating, according to the received sleep information of the user and environment information of the sleep environment of the user, a corresponding environment control instruction, and sending the environment control instruction to a local server;
after receiving the environment control instruction, the local server sending the environment control instruction to a corresponding adjustor; and
the adjustor controlling and regulating, according to the received environment control instruction, the illumination intensity of an LED lamp, the volume of an audio player and the rotational speed of a fan to realize an adjustment of the light, the sound and the airflow velocity of the sleep environment of the user, so as to realize an adjustment of the sleep environment of the user.

Further, the method further comprises the following step:
the adjusting server generating, according to the received sleep information of the user, a sleep data report of the user, and storing the sleep data report of the user in a database.

Further, before the data acquisition structure acquires sleep data of a user, the method further comprises the following steps:
the user manually inputting the illumination intensity of an LED lamp, the volume of an audio player and the rotational speed of a fan through a control panel;
the control panel outputting an adjustment instruction to an adjustor according to the illumination intensity of the LED lamp, the volume of the audio player and the rotational speed of the fan manually input by the user; and
the adjustor controlling and regulating, according to the received adjustment instruction, the illumination intensity of the LED lamp, the volume of the audio player and the rotational speed of the fan to realize the first adjustment of the light, the sound and the airflow velocity of the sleep environment of the user, so as to realize the first adjustment of the sleep environment of the user.

Further, the sleep data of the user includes the heart rate, body movement and/or respiratory rate of the user; the environment data of the sleep environment of the user includes the noise level and air temperature and humidity of the sleep environment of the user.

Further, the sleep information of the user includes the sleep time, wake-up time and sleep stage of the user; the environment information of the sleep environment of the user includes the noise level and air temperature and humidity of the sleep environment of the user.

According to the second aspect of the present invention, a system for adjusting a sleep environment is provided, comprising:
a data acquisition structure, used for acquiring data of user sleep and sending same to a data analysis server, the data of the user sleep comprising sleep data of a user and environment data of a sleep environment of a user;
the data analysis server, used for analyzing and processing the received data of the user sleep to obtain sleep information of a user and environment information of a sleep environment of the user, and sending the sleep information of the user and the environment information of the sleep environment of the user to an adjusting server;
the adjusting server, used for generating, according to the received sleep information of the user and environment information of the sleep environment of the user, a corresponding environment control instruction, and sending the environment control instruction to a local serve;
the local server, used for sending the environment control instruction to a corresponding adjustor after receiving the environment control instruction; and
the adjustor, used for controlling and regulating, according to the received environment control instruction, the illumination intensity of an LED lamp, the volume of an audio player and the rotational speed of a fan to realize an adjustment of the light, the sound and the airflow velocity of the sleep environment of the user, so as to realize an adjustment of the sleep environment of the user.

Further, the adjusting server is further used for generating, according to the received sleep information of the user, a sleep data report of the user, and storing the sleep data report of the user in a database.

Further, the system further comprises a control panel, used for manually inputting the illumination intensity of an LED lamp, the volume of an audio player and the rotational speed of a fan by the user and for outputting an adjustment instruction to an adjustor according to the illumination intensity of the LED lamp, the volume of the audio player and the rotational speed of the fan manually input by the user; the adjustor is further used for controlling and regulating, according to the received adjustment instruction, the illumination intensity of the LED lamp, the volume of the audio player and the rotational speed of the fan to realize the first adjustment of the light, the sound and the airflow velocity of the sleep environment of the user, so as to realize the first adjustment of the sleep environment of the user.

Further, the sleep data of the user includes the heart rate, body movement and/or respiratory rate of the user; the environment data of the sleep environment of the user includes the noise level and air temperature and humidity of the sleep environment of the user.

Further, the sleep information of the user includes the sleep time, wake-up time and sleep stage of the user; the environment information of the sleep environment of the user includes the noise level and air temperature and humidity of the sleep environment of the user.

According to the third aspect of the present invention, an apparatus for adjusting a sleep environment is provided, which comprises a data acquisition structure, an adjustor, an LED lamp, an audio player and a fan, the LED lamp, the audio player and the fan are connected to the adjustor, respectively; the data acquisition structure is used for acquiring data of user sleep and sends same to a data analysis server, and the data of the user sleep comprises sleep data of a user and environment data of a sleep environment of a user, and the adjustor is used for receiving an environment control instruction sent by a server and controlling and regulating, according to the environment control instruction, the illumination intensity of the LED lamp, the volume of the audio player and the rotational speed of the fan to realize an adjustment of the light, the sound and the airflow velocity of the sleep environment of the user, so as to realize an adjustment of the sleep environment of the user.

Further, the adjustor comprises a housing, and a processing module, a power module and a communication module arranged inside the housing, the power module and the communication module are connected to the processing module, respectively, a power interface is arranged on the housing and connected to the power module; the audio player is arranged inside the housing, and a through hole corresponding to the audio player is arranged on the housing; the LED lamp, the audio player and the fan are all connected to the processing module and the power module.

Further, the power module is provided with a first voltage conversion unit and a second voltage conversion unit, the processing module, the LED lamp and the audio player are connected to the first voltage conversion unit, respectively, the first voltage conversion unit is used for converting the supply voltage of the power module into the working voltage of the processing module, the LED lamp and the audio player, the fan is connected to the second voltage conversion unit, and the second voltage conversion unit is used for converting the supply voltage of the power module to the working voltage of the fan.

Further, the supply voltage of the power module is 220V, the working voltage of the processing module, the LED lamp and the audio player is 5V, and the working voltage of the fan is 12V

Further, a socket is arranged on the housing and connected to the power module.

Further, the adjusting device further comprises a control panel, and the control panel is connected to the processing module.

Further, the control panel comprises a master control module, and a lighting control module, a volume control module and a fan speed control module connected to the master control module, and the master control module is connected to the processing module.

Further, a data interface is arranged on the housing, and the master control module is connected to the processing module by means of the data interface.

Further, the communication module is a WIFI module or a wireless AP module.

Further, the data acquisition structure comprises a sleep sensor and an environmental sensor, the sleep sensor is used for acquiring sleep data of a user, the sleep data of the user includes the heart rate, body movement and/or respiratory rate of the user, the environmental sensor is used for acquiring environment data of a sleep environment of the user, the environment data of the sleep environment of the user includes the noise level and air temperature and humidity of the sleep environment of the user.

According to the present invention, sleep data of a user and environment data of a sleep environment of the user are acquired, analyzed and processed to obtain sleep information of the user and environment information of the sleep environment of the user, a corresponding environment control instruction is generated according to the sleep information of the user and the environment information of the sleep environment of the user, the illumination intensity of an LED lamp, the sound of an audio player and the rotational speed of a fan are regulated and controlled according to the environment control instruction to realize an adjustment of the light, the sound and the airflow velocity of the sleep environment of the user, so as to realize an adjustment of the sleep environment of the user, ensure the comfort of the user's sleep and realize the high-quality sleep of the user.

### [BRIEF DESCRIPTION OF THE DRAWINGS]

Fig. 1 is a block diagram of a system for adjusting a sleep environment provided by Embodiment 1 of the present invention;
Fig. 2 is a process diagram of a method for adjusting a sleep environment provided by Embodiment 2 of the present invention;
Fig. 3 is a block diagram of an apparatus for adjusting a sleep environment provided by Embodiment 3 of the present invention;
Fig. 4 is a structural schematic view of the adjusting device shown in Fig. 3 (the adjustor is an exploded view);
Fig. 5 is a block diagram of the adjustor shown in Fig. 4;
Fig. 6 is a block diagram of the control panel of the adjusting device shown in Fig. 3.

### [DETAILED DESCRIPTION]

The present invention is further described below in conjunction with the drawings and embodiments.

### Embodiment 1

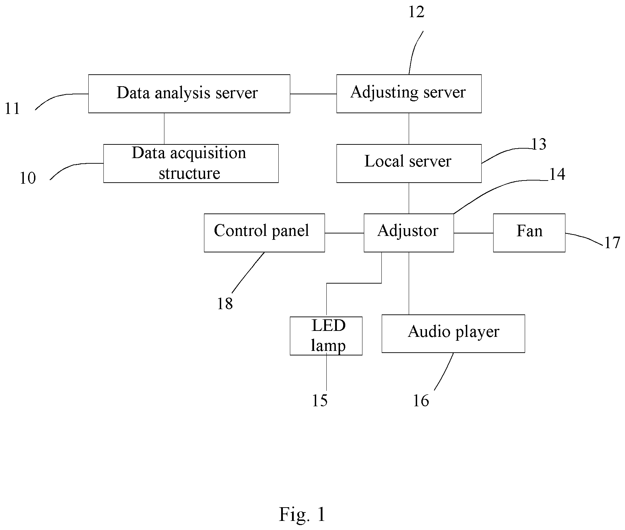

As shown in Fig. 1, a system for adjusting a sleep environment provided by the present invention comprises a data acquisition structure 10, a data analysis server 11, an adjusting server 12, a local server 13, an adjustor 14, an LED lamp 15, an audio player 16, a fan 17 and a control panel 18.

The data acquisition structure 10 is used for acquiring data of user sleep and sending same to a data analysis server 11. The data of the user sleep comprise sleep data of a user and environment data of a sleep environment of a user. The sleep data of the user includes the heart rate, body movement (i.e., the turnover movement of the user's body) and/or respiratory rate of the user; the environment data of the sleep environment of the user includes the noise level and air temperature and humidity of the sleep environment of the user.

The data analysis server 11 is used for analyzing and processing the received data of the user sleep to obtain sleep information of a user and environment information of a sleep environment of the user, and sending the sleep information of the user and the environment information of the sleep environment of the user to an adjusting server 12. The sleep information of the user includes the sleep time, wake-up time and sleep stage of the user; the sleep stage is a light sleep stage or a deep sleep stage for example. The environment information of the sleep environment of the user includes the noise level and air temperature and humidity of the sleep environment of the user.

The adjusting server 12 is used for generating, according to the received sleep information of the user and environment information of the sleep environment of the user, a corresponding environment control instruction, and sending the environment control instruction to a local serve 13. For example, the adjusting server 12 generates, according to the received sleep time, wake-up time and sleep stage of the user, an environment control instruction for turning off, up, down or on, and generates, according to the noise level and air temperature and humidity of the sleep environment of the user, an environment control instruction for turning high or low.

The local server 13 is used for sending the environment control instruction to a corresponding adjustor 14 after receiving the environment control instruction.

The adjustor 14 is used for controlling and regulating, according to the received environment control instruction, the illumination intensity of an LED lamp 15, the volume of an audio player 16 and the rotational speed of a fan 17 to realize an adjustment of the light, the sound and the airflow velocity of the sleep environment of the user, so as to realize an adjustment of the sleep environment of the user. The adjustor 14 corresponds to the data acquisition structure 10. For example, if the adjustor 14 receives an environment control instruction for turning off, it means that it is time for sleep of the user, so the adjustor 14 controls and regulates the illumination intensity of the LED lamp 15 to an illumination intensity that promotes the user to fall asleep, controls and regulates the volume of the audio player 16 to a volume that promotes the user to fall asleep, and controls and regulates the rotational speed of the fan 17 to a rotational speed that promotes the user to fall asleep, this circumstance helps the user fall asleep; if the adjustor 14 receives an environment control instruction for turning on, it means that it is time for wake of the user, so the adjustor 14 controls and regulates the illumination intensity of the LED lamp 15 to a certain value to turns on the LED lamp 15, controls and regulates the volume of the audio player 16 to a certain value to turns on the audio player 16, and controls and regulates the rotational speed of the fan 17 to a certain value to turns on the fan 17, this circumstance helps the user to wake up; if the adjustor 14 receives an environment control instruction for turning up, it means that the user is in a light sleep stage, so the adjustor 14 controls and regulates the illumination intensity of the LED lamp 15 to 0 to turns off the LED lamp 15, controls and regulates the volume of the audio player 16 to 0 to turns off the audio player 16, and controls and regulates the rotational speed of the fan 17 to increase, this circumstance helps the user to enter deep sleep from light sleep. Because people's body temperature will rise after they fall asleep, while a cool environment will be conducive to people's sleep, it is necessary to increase the rotational speed of the fan 17, so as to reduce the ambient temperature of user sleep, thereby helping the user enter a deep sleep from a light sleep; if the adjustor 14 receives an environment control instruction for turning down, it means that the user is in a deep sleep stage, so the adjustor 14 controls and regulates the illumination intensity of the LED lamp 15 to 0 to turns off the LED lamp 15, controls and regulates the volume of the audio player 16 to 0 to turns off the audio player 16, and controls and regulates the rotational speed of the fan 17 to decrease. For another example, if the adjustor 14 receives an environment control instruction for turning high, it means that the noise is large and the air temperature and humidity are high in the sleep environment of the user, so the adjustor 14 controls and regulates the illumination intensity of the LED lamp 15 to decrease, the volume of the audio player 16 to increase, and the rotational speed of the fan 17 to increase; if the adjustor 14 receives an environment control instruction for turning low, it means that the noise is small and the air temperature and humidity are low in the sleep environment of the user, so the adjustor 14 controls and regulates the illumination intensity of the LED lamp 15 to increase, the volume of the audio player 16 to decrease, and the rotational speed of the fan 17 to decrease.

The data acquisition structure 10 preferably is connected to the data analysis server 11 by means of a local router to send the acquired data of user sleep to the data analysis server 11. The adjusting server 12 preferably sends an environment control instruction to the local server 13 by means of the local router. The adjustor 14 is connected to the local server 13 by means of a wireless network, for example, WIFI or wireless AP. The LED lamp 15, the audio player 16 and the fan 17 are connected to the adjustor 14, respectively. Preferably, the audio player 16 is a loudspeaker, arranged at the bottom of the adjustor 14.

In this embodiment, the adjusting server 12 is further used for generating, according to the received sleep information of the user, a sleep data report of the user, and storing the sleep data report of the user in a database, so that the user can know his/her own sleep quality.

The control panel 18 is used for manually inputting the illumination intensity of an LED lamp 15, the volume of an audio player 16 and the rotational speed of a fan 17 by the user and for outputting an adjustment instruction to an adjustor 14 according to the illumination intensity of the LED lamp, the volume of the audio player and the rotational speed of the fan manually input by the user. The adjustor 14 is further used for controlling and regulating, according to the received adjustment instruction, the illumination intensity of the LED lamp 15, the volume of the audio player 16 and the rotational speed of the fan 17 to realize the first adjustment of the light, the sound and the airflow velocity of the sleep environment of the user, so as to realize the first adjustment of the sleep environment of the user.

### Embodiment 2

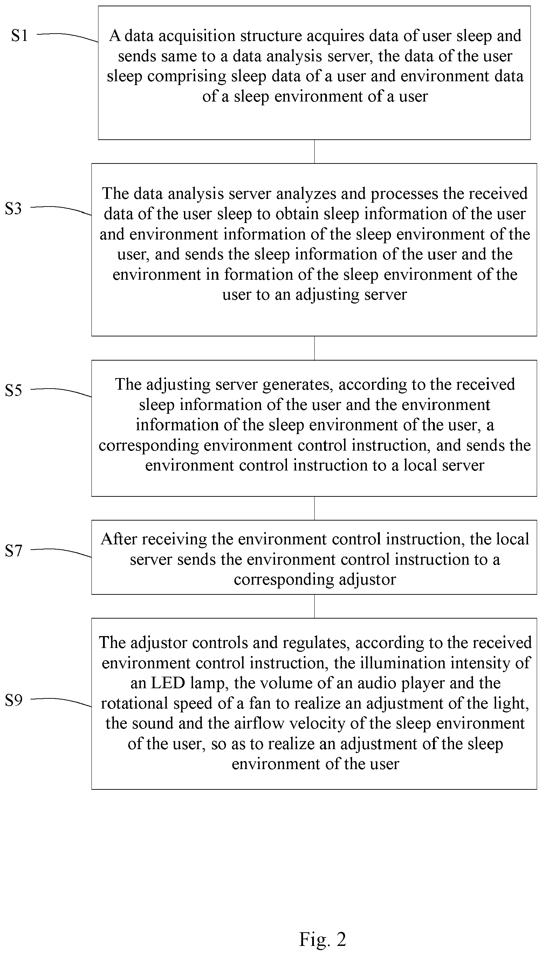

As shown in Fig. 2, this embodiment is a method for adjusting a sleep environment provided based on Embodiment 1, comprising the following steps:
S1, a data acquisition structure 10 acquiring data of user sleep and sending same to a data analysis server 11, the data of the user sleep comprising sleep data of a user and environment data of a sleep environment of a user. Preferably, the data acquisition structure 10 sends the acquired data of user sleep to the data analysis server 11 by means of a local router.
S3, the data analysis server 11 analyzing and processing the received data of the user sleep to obtain sleep information of a user and environment information of a sleep environment of the user, and sending the sleep information of the user and the environment information of the sleep environment of the user to an adjusting server 12.
S5, the adjusting server 12 generating, according to the received sleep information of the user and environment information of the sleep environment of the user, a corresponding environment control instruction, and sending the environment control instruction to a local server 13.
S7, after receiving the environment control instruction, the local server 13 sending the environment control instruction to a corresponding adjustor 14.
S9, the adjustor 14 controlling and regulating, according to the received environment control instruction, the illumination intensity of an LED lamp 15, the volume of an audio player 16 and the rotational speed of a fan 17 to realize an adjustment of the light, the sound and the airflow velocity of the sleep environment of the user, so as to realize an adjustment of the sleep environment of the user.

Further, the method further comprises the following step: the adjusting server 12 generating, according to the received sleep information of the user, a sleep data report of the user, and storing the sleep data report of the user in a database.

Further, before the data acquisition structure 10 acquires sleep data of a user, the method further comprises the following steps:
the user manually inputting the illumination intensity of an LED lamp 15, the volume of an audio player 16 and the rotational speed of a fan 17 through a control panel 18.
the control panel 18 outputting an adjustment instruction to an adjustor 14 according to the illumination intensity of the LED lamp 15, the volume of the audio player 16 and the rotational speed of the fan 17 manually input by the user.
the adjustor 14 controlling and regulating, according to the received adjustment instruction, the illumination intensity of the LED lamp 15, the volume of the audio player 16 and the rotational speed of the fan 17 to realize the first adjustment of the light, the sound and the airflow velocity of the sleep environment of the user, so as to realize the first adjustment of the sleep environment of the user.

In this embodiment, the sleep data of the user includes the heart rate, body movement (i.e., the turnover movement of the user's body) and/or respiratory rate of the user; the environment data of the sleep environment of the user includes the noise level and air temperature and humidity of the sleep environment of the user.

The sleep information of the user includes the sleep time, wake-up time and sleep stage of the user; the environment information of the sleep environment of the user includes the noise level and air temperature and humidity of the sleep environment of the user.

To sum up, according to the present invention, sleep data of a user and environment data of a sleep environment of the user are acquired, analyzed and processed to obtain sleep information of the user and environment information of the sleep environment of the user, an environment control instruction is generated according to the sleep information of the user and the environment information of the sleep environment of the user, the illumination intensity of an LED lamp 15, and the sound of an audio player 16 and the rotational speed of a fan 17 are regulated and controlled according to the environment control instruction to realize an adjustment of the light, the sound and the airflow velocity of the sleep environment of the user, so as to realize an adjustment of the sleep environment of the user, ensure the comfort of the user's sleep and realize the high-quality sleep of the user.

### Embodiment 3

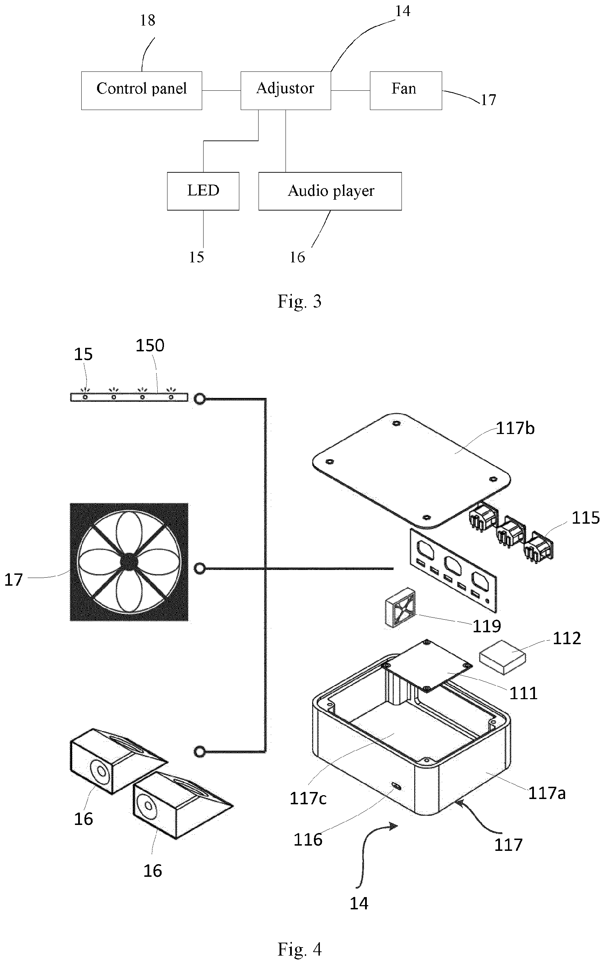

As shown in Fig. 3, this embodiment is an apparatus for adjusting a sleep environment provided based on Embodiment 1, and used for being mounted inside a rest device. The apparatus comprises a data acquisition structure (not shown in the figure), an adjustor 14, an LED lamp 15, an audio player 16 and a fan 17. The LED lamp 15, the audio player 16 and the fan 17 are connected to the adjustor 14, respectively. The data acquisition structure is used for acquiring data of user sleep and sending same to a server (i.e., a data analysis server). The data of the user sleep comprises sleep data of a user and environment data of a sleep environment of a user. Preferably, the data acquisition structure comprises a sleep sensor and an environmental sensor. The sleep sensor is mounted at bed bottom inside a rest device in general and used for acquiring sleep data of a user, and the sleep data of the user includes the heart rate, body movement (i.e., the turnover movement of the user's body) and/or respiratory rate of the user. The environmental sensor is mounted on a wall inside the rest device and used for acquiring environment data of a sleep environment of the user, and the environment data of the sleep environment of the user includes the noise level and air temperature and humidity of the sleep environment of the user. The adjustor 14 is used for receiving an environment control instruction sent by a server (i.e., a local server) and controlling and regulating, according to the environment control instruction, the illumination intensity of the LED lamp 15, the volume of the audio player 16 and the rotational speed of the fan 17 to realize an adjustment of the light, the sound and the airflow velocity of the sleep environment of the user, so as to realize an adjustment of the sleep environment of the user.

For example, if the adjustor 14 receives an environment control instruction for turning off, it means that it is time for sleep of the user, so the adjustor 14 controls and regulates the illumination intensity of the LED lamp 15 to an illumination intensity that promotes the user to fall asleep, controls and regulates the volume of the audio player 16 to a volume that promotes the user to fall asleep, and controls and regulates the rotational speed of the fan 17 to a rotational speed that promotes the user to fall asleep, this circumstance helps the user fall asleep; if the adjustor 14 receives an environment control instruction for turning on from the server, it means that it is time for wake of the user, so the adjustor 14 controls and regulates the illumination intensity of the LED lamp 15 to a certain value to turns on the LED lamp 15, controls and regulates the volume of the audio player 16 to a certain value to turns on the audio player 16, and controls and regulates the rotational speed of the fan 17 to a certain value to turns on the fan 17, this circumstance helps the user to wake up; if the adjustor 14 receives an environment control instruction for turning up from the server, it means that the user is in a light sleep stage, so the adjustor 14 controls and regulates the illumination intensity of the LED lamp 15 to 0 to turns off the LED lamp 15, controls and regulates the volume of the audio player 16 to 0 to turns off the audio player 16, and controls and regulates the rotational speed of the fan 17 to increase, this circumstance helps the user to enter deep sleep from light sleep. Because people's body temperature will rise after they fall asleep, while a cool environment will be conducive to people's sleep, it is necessary to increase the rotational speed of the fan 17, so as to reduce the ambient temperature of user sleep, thereby helping the user enter a deep sleep from a light sleep; if the adjustor 14 receives an environment control instruction for turning down from the server, it means that the user is in a deep sleep stage, so the adjustor 14 controls and regulates the illumination intensity of the LED lamp 15 to 0 to turns off the LED lamp 15, the volume of the audio player 16 to 0 to turns off the audio player 16, and controls and regulates the rotational speed of the fan 17 to decrease. For another example, if the adjustor 14 receives an environment control instruction for turning high from the server, it means that the noise is large and the air temperature and humidity are high in the sleep environment of the user, so the adjustor 14 controls and regulates the illumination intensity of the LED lamp 15 to decrease, the volume of the audio player 16 to increase, and the rotational speed of the fan 17 to increase; if the adjustor 14 receives an environment control instruction for turning low from the server, it means that the noise is small and the air temperature and humidity are low in the sleep environment of the user, so the adjustor 14 controls and regulates the illumination intensity of the LED lamp 15 to increase, the volume of the audio player 16 to decrease, and the rotational speed of the fan 17 to decrease.

Preferably, there are several LED lamps 15. The several LED lamps 15 form a light strip 150. The audio player 16 preferably is a loudspeaker. There are two audio players 16. The number of the LED lamps 15 and the audio players 16 can be set according to the actual condition.

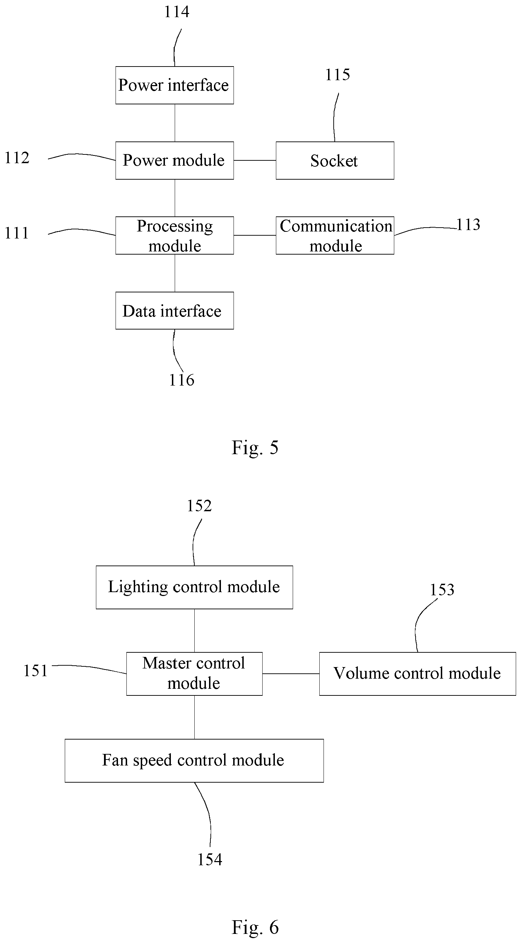

As shown in Fig. 4 and Fig. 5, specifically, the adjustor 14 comprises a square housing 117, and a processing module 111, a power module 112 and a communication module 113 arranged inside the housing 117. The processing module 111 preferably is a CPU (central processing unit). The power module 112 is located on a side in the housing 117. The power module 112 and the communication module 113 are connected to the processing module 111, respectively. A power interface 114 is arranged on a side of housing 117 and in a position corresponding to the power module 112, and is connected to the power module 112. The power module 112 preferably is a mobile power supply. The power interface 114 preferably is a Type-C interface, USB2.0 interface or USB3.0 interface and is used for connecting an external power supply to charge the power module 112. The top of the housing 117 is provided with a power switch, and the power switch is connected to the processing module 111 and used for controlling the connection and disconnection of the power supply. The audio player 16 is arranged inside the housing 117, and a through hole corresponding to the audio player 16 is arranged on the housing 117. Preferably, the audio player 16 is arranged at the inner bottom of the housing 117, and a through hole corresponding to the audio player 16 is arranged on the inner bottom of the housing 117. The LED lamp 15, the audio player 16 and the fan 17 are all connected to the processing module 111 and the power module 112. The power module 112 supplies power to the processing module 111, the LED lamp 15, the audio player 16 and the fan 17, respectively. The processing module 111 is used for controlling and regulating the illumination intensity of the LED lamp 15, the volume of the audio player 16 and the rotational speed of the fan 17 to realize an adjustment of the light, the sound and the airflow velocity of the sleep environment of the user.

The communication module 113 is a wireless AP module. After the adjustor 14 is powered on, the wireless AP module will serve as an AP hotspot. After a user uses a smart terminal, a mobile phone for example, to access the AP hotspot, the mobile phone is hooked to the Internet. After the mobile phone is successfully hooked to the Internet, the adjustor 14 is connected to the Internet. Thus, environment control instructions sent by a server can be received. It can be understood that the communication module 113 can be a WIFI module, too, and through the WIFI module, the Internet is accessed to realize reception of environment control instructions sent by a server.

In this embodiment, the power module 112 is provided with a first voltage conversion unit and a second voltage conversion unit. The processing module 111, the LED lamp 15 and the audio player 16 are connected to the first voltage conversion unit, respectively, the first voltage conversion unit is used for converting the supply voltage of the power module 112 into the working voltage of the processing module 111, the LED lamp 15 and the audio player 16. The fan 17 is connected to the second voltage conversion unit, and the second voltage conversion unit is used for converting the supply voltage of the power module 112 to the working voltage of the fan 17. Preferably, the supply voltage of the power module 112 is 220V, and the working voltage of processing module 111, the LED lamp 5 and the audio player 16 is 5V, and the working voltage of the fan 17 is 12V.

Further, a socket 115 is arranged on the housing 117 and connected to the power module 112. The socket 115 can be used for charging of other electronic devices. In this embodiment, there are three sockets 115. The number of the sockets 115 can be set according to the actual condition. The sockets 115 preferably are three-pin sockets. It can be understood that the sockets 115 can be other types of sockets, too.

Further, a cooling fan 119 connected to the processing module 111 is arranged inside the housing 117 and used for heat dissipation.

In this embodiment, the housing 117 comprises a housing body 117a with openings at the two ends, and a top plate 117b and a bottom plate 117c arranged at the two ends of the housing body 117a. The processing module 111, the power module 112 and the communication module 113 are arranged inside the housing body 117a, the power interface 114 and the socket 115 are arranged on the same side of the housing body 117a, the audio player 16 and the through hole are arranged on the bottom plate 117c, and the power switch is arranged on the top plate 117b.

As shown in Fig. 3 and Fig. 6, the adjusting device further comprises a control panel 18, and the control panel 18 is connected to the processing module 111. The control panel 18 is used for sending an adjustment instruction to the processing module 111, and the processing module 111 is used for controlling and regulating the illumination intensity of the LED lamp 15, the volume of the audio player 16 and the rotational speed of the fan 17 according to the adjustment instruction.

Specifically, the control panel 18 comprises a master control module 151, and a lighting control module 152, a volume control module 153 and a fan speed control module 154 connected to the master control module 151. The master control module 151 is connected to the processing module 111. The lighting control module 152 preferably is a lighting control button. Through the lighting control button, the illumination intensity of the LED lamp 15 can be set manually. The volume control module 153 preferably is a volume control button. Through the volume control button, the volume of the audio player 16 can be set manually. The fan speed control module 154 preferably is a fan speed control button. Through the fan speed control button, the rotational speed of the fan 17 can be set manually. After the illumination intensity of the LED lamp 15, the volume of the audio player 16 and the rotational speed of the fan 17 are set, the control panel 18 outputs an adjustment instruction to the processing module 11. After receiving the adjustment instruction, the processing module 111 can control and regulate the illumination intensity of the LED lamp 15, the volume of the audio player 16 and the rotational speed of the fan 17. On the control panel 18, the illumination intensity of the LED lamp 15, the volume of the audio player 16 and the rotational speed of the fan 17 are set manually, before user sleep in general, so as to realize the first adjustment to the sleep environment of the user. Through this structure, the user can manually set the illumination intensity of the LED lamp 15, the volume of the audio player 16 and the rotational speed of the fan 17 according to his/her own condition.

Further, a data interface 116 is arranged on the housing 227 (see Fig. 4 and Fig. 5), the master control module 151 is connected to the processing module 111 by means of the data interface 116. Preferably, the data interface 116 and the power interface 114 are respectively arranged on two opposite sides of the housing body 117a. The data interface 116 is a USB interface for example.

To sum up, through the data acquisition structure and the adjustor 14, the present invention can control and regulate the illumination intensity of the LED lamp 15, the volume of the audio player 16 and the rotational speed of the fan 17 to realize an adjustment of the light, the sound and the airflow velocity of the sleep environment, so as to realize an adjustment of the sleep environment of the user, ensure the comfort of the user's sleep and realize the high-quality sleep of the user.

The foregoing embodiments only represent the preferred embodiments of the present invention. Their descriptions are concrete and detailed, but they shall not be therefore understood as limitations to the scope of the present invention patent. It shall be noted that for those skilled in the art, various changes and modifications may be made to the embodiments without departing from the spirit of the present invention, such as: combinations of different features of the embodiments. All these shall be in the protective scope of the present invention.

## Claims

1. A method for adjusting a sleep environment, wherein the method comprises the following steps:
a data acquisition structure acquiring data of user sleep and sending same to a data analysis server, the data of the user sleep comprising sleep data of a user and environment data of a sleep environment of a user;
the data analysis server analyzing and processing the received data of the user sleep to obtain sleep information of a user and environment information of a sleep environment of the user, and sending the sleep information of the user and the environment information of the sleep environment of the user to an adjusting server;
the adjusting server generating, according to the received sleep information of the user and environment information of the sleep environment of the user, a corresponding environment control instruction, and sending the environment control instruction to a local server;
after receiving the environment control instruction, the local server sending the environment control instruction to a corresponding adjustor; and
the adjustor controlling and regulating, according to the received environment control instruction, the illumination intensity of an LED lamp, the volume of an audio player and the rotational speed of a fan to realize an adjustment of the light, the sound and the airflow velocity of the sleep environment of the user, so as to realize an adjustment of the sleep environment of the user.

2. The method for adjusting a sleep environment according to claim 1, wherein the method further comprises the following step:
the adjusting server generating, according to the received sleep information of the user, a sleep data report of the user, and storing the sleep data report of the user in a database.

3. The method for adjusting a sleep environment according to claim 1, wherein before the data acquisition structure acquires sleep data of a user, the method further comprises the following steps:
the user manually inputting the illumination intensity of an LED lamp, the volume of an audio player and the rotational speed of a fan through a control panel;
the control panel outputting an adjustment instruction to an adjustor according to the illumination intensity of the LED lamp, the volume of the audio player and the rotational speed of the fan manually input by the user; and
the adjustor controlling and regulating, according to the received adjustment instruction, the illumination intensity of the LED lamp, the volume of the audio player and the rotational speed of the fan to realize the first adjustment of the light, the sound and the airflow velocity of the sleep environment of the user, so as to realize the first adjustment of the sleep environment of the user.

4. The method for adjusting a sleep environment according to claim 1, wherein the sleep data of the user includes the heart rate, body movement and/or respiratory rate of the user; the environment data of the sleep environment of the user includes the noise level and air temperature and humidity of the sleep environment of the user.

5. The method for adjusting a sleep environment according to claim 4, wherein the sleep information of the user includes the sleep time, wake-up time and sleep stage of the user; the environment information of the sleep environment of the user includes the noise level and air temperature and humidity of the sleep environment of the user.

6. A system for adjusting a sleep environment, wherein the system comprises:
a data acquisition structure, used for acquiring data of user sleep and sending same to a data analysis server, the data of the user sleep comprising sleep data of a user and environment data of a sleep environment of a user;
the data analysis server, used for analyzing and processing the received data of the user sleep to obtain sleep information of a user and environment information of a sleep environment of the user, and sending the sleep information of the user and the environment information of the sleep environment of the user to an adjusting server;
the adjusting server, used for generating, according to the received sleep information of the user and environment information of the sleep environment of the user, a corresponding environment control instruction, and sending the environment control instruction to a local serve;
the local server, used for sending the environment control instruction to a corresponding adjustor after receiving the environment control instruction; and
the adjustor, used for controlling and regulating, according to the received environment control instruction, the illumination intensity of an LED lamp, the volume of an audio player and the rotational speed of a fan to realize an adjustment of the light, the sound and the airflow velocity of the sleep environment of the user, so as to realize an adjustment of the sleep environment of the user.

7. The system for adjusting a sleep environment according to claim 6, wherein the adjusting server is further used for generating, according to the received sleep information of the user, a sleep data report of the user, and storing the sleep data report of the user in a database.

8. The system for adjusting a sleep environment according to claim 7, wherein the system further comprises a control panel, used for manually inputting the illumination intensity of an LED lamp, the volume of an audio player and the rotational speed of a fan by the user and for outputting an adjustment instruction to an adjustor according to the illumination intensity of the LED lamp, the volume of the audio player and the rotational speed of the fan manually input by the user; the adjustor is further used for controlling and regulating, according to the received adjustment instruction, the illumination intensity of the LED lamp, the volume of the audio player and the rotational speed of the fan to realize the first adjustment of the light, the sound and the airflow velocity of the sleep environment of the user, so as to realize the first adjustment of the sleep environment of the user.

9. The system for adjusting a sleep environment according to claim 6, wherein the sleep data of the user includes the heart rate, body movement and/or respiratory rate of the user; the environment data of the sleep environment of the user includes the noise level and air temperature and humidity of the sleep environment of the user.

10. The system for adjusting a sleep environment according to claim 9, wherein the sleep information of the user includes the sleep time, wake-up time and sleep stage of the user; the environment information of the sleep environment of the user includes the noise level and air temperature and humidity of the sleep environment of the user.

11. An apparatus for adjusting a sleep environment, wherein the apparatus comprises a data acquisition structure, an adjustor, an LED lamp, an audio player and a fan, and the LED lamp, the audio player and the fan are connected to the adjustor, respectively; the data acquisition structure is used for acquiring data of user sleep and sending same to a data analysis server, the data of the user sleep comprises sleep data of a user and environment data of a sleep environment of a user, the adjustor is used for receiving an environment control instruction sent by a server and controlling and regulating, according to the environment control instruction, the illumination intensity of the LED lamp, the volume of the audio player and the rotational speed of the fan to realize an adjustment of the light, the sound and the airflow velocity of the sleep environment of the user, so as to realize an adjustment of the sleep environment of the user.

12. The apparatus for adjusting a sleep environment according to claim 11, wherein the adjustor comprises a housing, and a processing module, a power module and a communication module arranged inside the housing, the power module and the communication module are connected to the processing module, respectively, and a power interface is arranged on the housing and connected to the power module; the audio player is arranged inside the housing, and a through hole corresponding to the audio player is arranged on the housing; the LED lamp, the audio player and the fan are all connected to the processing module and the power module.

13. The apparatus for adjusting a sleep environment according to claim 12, wherein the power module is provided with a first voltage conversion unit and a second voltage conversion unit, the processing module, the LED lamp and the audio player are connected to the first voltage conversion unit, respectively, the first voltage conversion unit is used for converting the supply voltage of the power module into the working voltage of the processing module, the LED lamp and the audio player, the fan is connected to the second voltage conversion unit, and the second voltage conversion unit is used for converting the supply voltage of the power module to the working voltage of the fan.

14. The apparatus for adjusting a sleep environment according to claim 13, wherein the supply voltage of the power module is 220V, the working voltage of the processing module, the LED lamp and the audio player is 5V, and the working voltage of the fan is 12V.

15. The apparatus for adjusting a sleep environment according to claim 12, wherein a socket is arranged on the housing and connected to the power module.

16. The apparatus for adjusting a sleep environment according to claim 12, wherein the adjusting device further comprises a control panel, and the control panel is connected to the processing module.

17. The apparatus for adjusting a sleep environment according to claim 16, wherein the control panel comprises a master control module, and a lighting control module, a volume control module and a fan speed control module connected to the master control module, and the master control module is connected to the processing module.

18. The apparatus for adjusting a sleep environment according to claim 17, wherein a data interface is arranged on the housing, and the master control module is connected to the processing module by means of the data interface.

19. The apparatus for adjusting a sleep environment according to claim 12, wherein the communication module is a WIFI module or a wireless AP module.

20. The apparatus for adjusting a sleep environment according to claim 11, wherein the data acquisition structure comprises a sleep sensor and an environmental sensor, the sleep sensor is used for acquiring sleep data of a user, the sleep data of the user includes the heart rate, body movement and/or respiratory rate of the user, the environmental sensor is used for acquiring environment data of a sleep environment of the user, and the environment data of the sleep environment of the user includes the noise level and air temperature and humidity of the sleep environment of the user.
